# EUROPEAN PATENT APPLICATION

(11) **EP 2 277 859 A1**
(43) Date of publication of application: **26.01.2011**
(21) Application number: 09178834.9
(22) Date of filing: 11.12.2009
(51) Int. Cl.: C07C 237/46, C07C 231/02

(54) **Acetylation using reduced concentration of acetic acid anhydride for synthesizing non-ionic X-ray contrast agents**

(30) Priority: 21.07.2009 US 227089 P
(71) Applicant: GE Healthcare AS, 0401 Oslo (NO)
(72) Inventor: Haaland, Torfinn, N-4521, Spangereid (NO)
(74) Representative: Wulff, Marianne Weiby

(57) **Abstract**

This invention relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to acetylation of 5-amino-N, N'-bis(2,3-dihydroxypropyl)-2,4,6-triiodo-1,3-bcnzcncdicarboxamide ("Compound B") using a reagent mixture containing about 50 v/v % to about 85 v/v % acetic anhydride and about 15 v/v % to about 50 v/v % acetic acid as a reagent mixture for acetylation of 5-amino-N, N'-bis(2,3-dihydroxypropyl)-2,4,6-triiodo-1,3-benzenedicarboxamide ("Compound B"). Preferably, following the acetylation reaction, the reagent and solvent mixture is distilled for re-use.

## Description

### TECHNICAL FIELD

This invention relates generally to large-scale synthesis of non-ionic X-ray contrast agents. It further relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to acetylation of 5-amino-N, N'-bis(2,3-dihydroxypropyl)-2,4,6-triiodo-1,3-benzenedicarboxamide ("Compound B") using reduced concentration of acetic anhydride.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol") and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

In particular, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. To reduce the cost of the final product, it is critical to optimize each synthetic step. Even a small improvement in reaction design can lead to significant savings in a large scale production.

The instant improvement is directed to the acetylation step, where 5-amino-N,N'-bis(2,3-dihydroxypropyl)-2,4,6-triiodoisophthalamide (Compound B) is acetylated to produce 5-acetylamino-N,N'-bis(2,3-dihydroxypropyl)-2,4,6-triiodoisophthalamide (Compound A) using acetic anhydride as the acetylating reagent. According to the present invention, the concentration of acetic anhydride used in the acetylation reaction is significantly reduced.

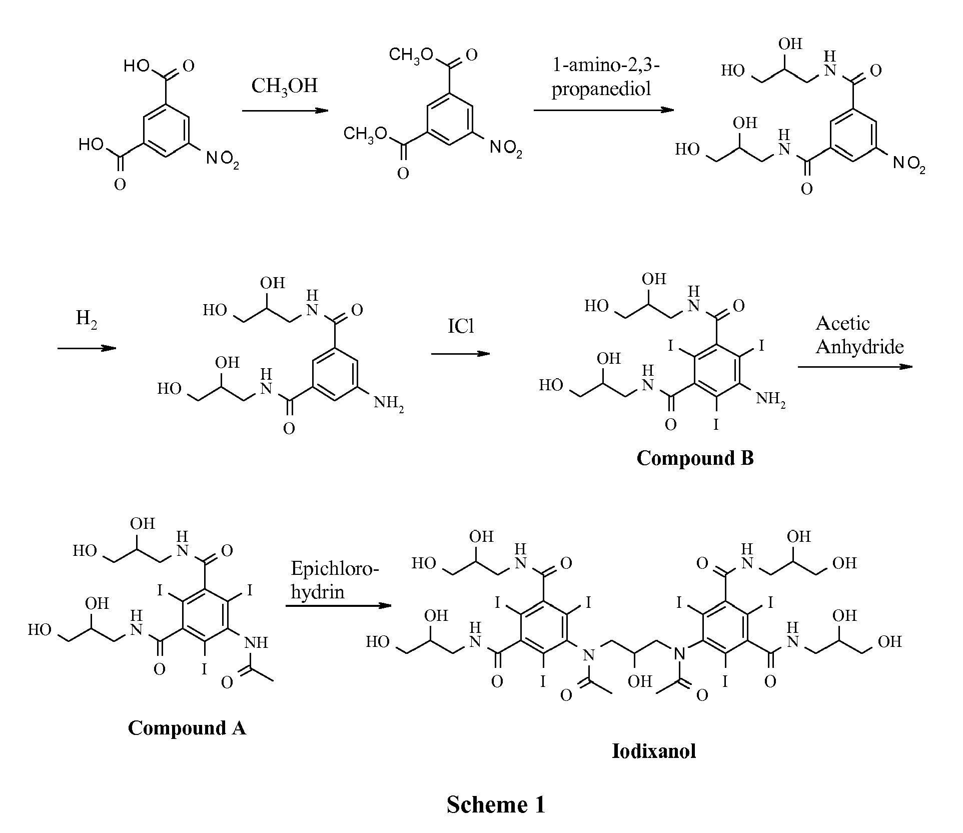

### SUMMARY OF THE INVENTION

The present invention provides an industrial process for preparing Compound A by acetylation of Compound B. Specifically, it uses about 50 v/v % to about 85 v/v % acetic anhydride and about 15 v/v % to about 50 v/v % acetic acid as a reagent mixture. In a preferred embodiment, following the acetylation reaction, the reagent and solvent mixture is distilled for re-use in a subsequent acetylation reaction from Compound B to Compound A.

### DETAILED DESCRIPTION OF THE INVENTION

In the acetylation reaction of Compound B, both the amino group and the hydroxyl groups are acetylated. While the hydroxyl groups are later deacetylated by the addition of aqueous sodium hydroxide, a full acetylation of Compound B involves the addition of six acetic anhydride equivalents (four O-acetyls and two N-acetyls from four hydroxyl groups and two amino hydrogens respectively). See Scheme 2. Traditionally, the lowest concentration of acetic anhydride in the reagent and solvent mixture of acetic anhydride and acetic acid used in the acetylation reaction has been about or over 90% (v/v).

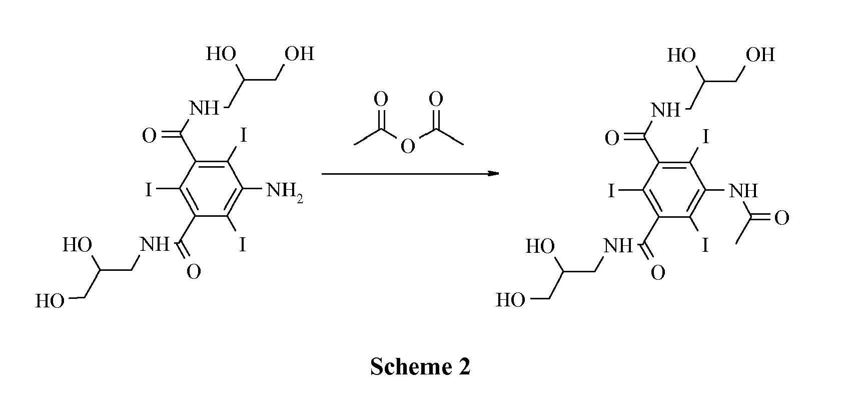

We have now surprisingly found that less concentration of acetic anhydride in the acetic anhydride and acetic acid mixture can be used in the acetylation reaction to allow for nearly full conversion from Compound B to Compound A. In particular, the necessary conversion of Compound B to Compound A can be obtained with between about 50 % (v/v) and about 85 % (v/v) of acetic anhydride, without increasing the total combined volume of acetic anhydride plus acetic acid.

It has been found that a total acetylation degree of at least 5.0 is required, since the hydroxyl groups are slightly more reactive than the amino group, and a full conversion to at least one aminoacetyl group is necessary to effect a full net conversion to Compound A after deacetylation. Specifically, in the deacetylation step, saponification of the acetoxy groups are more easily obtained than hydrolysis of the aminoacetyl group(s), making it possible to retain one aminoacetyl group unhydrolysed by optimizing the pH in the deacetylation step, thus obtaining Compound A in high purity. The deacetylation step may be performed after dilution of the process solution with methanol and water. Compound A may be crystallised from the deacetylated solution by the addition of hydrochloric acid followed by filtration, wash of the filter cake with a suitable solvent, e.g. methanol, and drying in a suitable dryer.

After the acetylation reaction excess acetic anhydride and acetic acid may be distilled for re-use. In particular, the distillation process may be performed under reduced pressure. Preferably, a fraction of the distillate may be re-used in the next batch of acetylation reaction and mixed with fresh acetic anhydride to form the desired concentration in the desired total amount of reagent and solvent. More preferably, the excess mixture of acetic anhydride and acetic acid is distilled and collected continuously. For example, earlier portions of the distillate have a much higher content of acetic acid than latter portions because acetic acid has a lower boiling point than acetic anhydride (118°C vs. 136°C at atmospheric pressure). As such, the latter portions of the distillate may be suitable for regeneration and re-use purposes because of their high acetic anhydride content. The split between non-useable and reusable distillate is determined by the mass balance for acetic anhydride and acetic acid, where the acetic acid content reaches a level where it is stoichiometrically impossible to obtain an acetylation degree of 5.0 (see above).

The instant invention provides several distinct advantages. First, the amount of expensive acetic anhydride consumed per batch is reduced. Because acetic acid is cheaper than acetic anhydride, the replacement of acetic anhydride with acetic acid lowers the reagent cost and in turn drives down the overall production cost for the drug substance. The cost of manufacture is further lowered because energy costs are reduced because the fraction of acetic acid vs. acetic anhydride in the distillate is significantly increased and acetic acid has a lower boiling point than acetic anhydride.

Second, acetic acid is easier to handle because it is much less reactive than acetic anhydride.

Finally, the fraction of distillate that is suitable for regeneration and re-use is drastically increased. Specifically, with more acetic acid content used in the acetylation reaction, more of the distillate can be re-used. This is because, as discussed above, earlier fractions of the distillate, which are rich in acetic acid, can be reused (without reaching the point where the mass balance disturbs the stoichiometry).

The invention is illustrated further by the following examples that are not to be construed as limiting the invention in scope to the specific procedures described in them.

### EXAMPLES

### EXAMPLE 1 (Comparative Example)

Acetic anhydride (240 mL) and regenerated acetic anhydride (containing about 50-55 v/v % acetic acid) (60 mL) were mixed in a reactor, giving a mixture containing 90 v/v % acetic anhydride and 10 v/v % acetic acid, which corresponds to 2.85 mole of acetic anhydride. Compound B (200 g, 0.28 mole) was added at ambient temperature and the mixture heated to 55°C. Then, *p*-toluene sulphonic acid (1.0 g, 0.0058 mole) was added and the mixture further heated to 120°C over one hour. The acetylation degree was measured by HPLC to be 5.41. In other words, on average, 5.41 of 6 potential groups that can be acetylated in each molecule of Compound B have been acetylated.

### EXAMPLE 2

Acetic anhydride (150 mL) and regenerated acetic anhydride (containing about 50-55 v/v % acetic acid) (150 mL) were mixed in a reactor, giving a mixture containing 75 v/v % acetic anhydride and 25 v/v % acetic acid, which corresponds to 2.38 mole of acetic anhydride. Compound B (200 g, 0.28 mole) was added at ambient temperature and the mixture heated to 55°C. Then, *p*-toluene sulphonic acid (1.0 g, 0.0058 mole) was added and the mixture further heated to 120°C over one hour. The acetylation degree was measured by HPLC to be 5.25. In other words, on average, 5.25 of 6 potential groups that can be acetylated in each molecule of Compound B have been acetylated.

### EXAMPLE 3

Acetic anhydride (40 mL) and regenerated acetic anhydride (containing about 50-55 v/v % acetic acid) (260 mL) were mixed in a reactor, giving a mixture containing 54 v/v % acetic anhydride and 46 v/v % acetic acid, which corresponds to 1.72 mole of acetic anhydride. Compound B (200 g, 0.28 mole) was added at ambient temperature and the mixture heated to 55°C. Then, *p*-toluene sulphonic acid (1.0 g, 0.0058 mole) was added and the mixture further heated to 120°C over one hour. The acetylation degree was measured by HPLC to be 5.04. In other words, on average, 5.04 of 6 potential groups that can be acetylated in each molecule of Compound B have been acetylated.

All patents, journal articles, publications and other documents discussed and/or cited above are hereby incorporated by reference.

## Claims

1. A process for industrial preparation of 5-acetamido-N,N'-bis(2,3-dihydroxypropyl)-2,4,6-triiodoisophthalamide ("Compound A") using about 50 % (v/v) to about 85 % (v/v) acetic anhydride and about 15 % (v/v) to about 50 % (v/v) acetic acid as a reagent and solvent mixture for acetylation of 5-amino-N, N'-bis(2,3-dihydroxypropyl)-2,4,6-triiodo-1,3-benzenedicarboxamide ("Compound B").

2. The process of claim 1 wherein the acetic anhydride and acetic acid solvent mixture is distilled, following the acetylation reaction, for re-use.
